# EUROPÄISCHE PATENTANMELDUNG

(11) **EP 4 691 424 A1**
(43) Veröffentlichungstag der Anmeldung: **11.02.2026**
(21) Anmeldenummer: 25190391.0
(22) Anmeldetag: 18.07.2025
(51) Int. Cl.: A61F 2/28, A61B 17/80

(54) **IMPLANTAT ZUM ERSATZ ODER AUFFÜLLEN EINER FEHLSTELLE EINES FLÄCHENARTIGEN KNOCHENS SOWIE VERFAHREN ZUR HERSTELLUNG EINES SOLCHEN IMPLANTATS**

(30) Priorität: 08.08.2024 DE 102024122716
(71) Anmelder: Karl Leibinger Asset Management GmbH & Co. KG, 78570 Mühlheim an der Donau (DE)
(72) Erfinder: Aksu, Adem, 78054 VS-Schwenningen (DE); Schaarschmidt, Lena, 78532 Tuttlingen (DE); Reinauer, Frank, 78576 Emmingen-Liptingen (DE); Wolfram, Tobias, 63303 Dreieich (DE)
(74) Vertreter: Schwamberger, Martin

(57) **Zusammenfassung**

Implantat (X) zum Ersatz oder Auffüllen einer Fehlstelle eines flächenartigen Knochens, wobei das Implantat (X) eine flächenartige Form mit einer ersten Seite (X1) und einer gegenüberliegenden zweiten Seite (X2) aufweist, wobei das Implantat (X) eine erste Schicht (S1) aus nicht-porösem Polyetheretherketon oder Polyethylen aufweist, welche eine solche Schichtdicke aufweist, dass die Form des Implantats (X) durch die Form der ersten Schicht (S1) festgelegt ist, wobei das Implantat (X) auf der ersten Seite (X1) zumindest abschnittsweise eine zweite Schicht (S2) aus einem porösen Polymer aufweist, welches kein Polyaryletherketon oder Polyetheretherketon ist, sowie Verfahren zur Herstellung eines solchen Implantats (X).

## Beschreibung

Die Erfindung betrifft ein Implantat zum Ersatz oder Auffüllen einer Fehlstelle eines flächenartigen Knochens, im menschlichen oder tierischen Körper. Die Erfindung betrifft ferner ein Verfahren zur Herstellung eines solchen Implantats.

Im Stand der Technik sind Implantate bekannt, die einen flächenartigen Knochen vollständig ersetzen, oder zum Auffüllen einer Fehlstelle eines solchen Knochens vorgesehen sind. Flächenartigen Knochen sind beispielsweise Schädelknochen, Rippen, Schulterblatt, oder Beckenknochen. So lehrt beispielsweise die WO 2015/144772 A1 ein Augenhöhlenabdeckgitter zum Ersatz eines Augenhöhlen-Abschnitts. Derartige Implantate müssen mechanisch stabil sein, sodass sie häufig aus einem biokompatiblen Metall gefertigt sind, beispielsweise Titan. Da die menschliche Anatomie individuell unterschiedlich ist, werden solche Implantate häufig patientenspezifisch gefertigt, und/oder werden intraoperativ in die korrekte Form gebracht, z.B. von Hand gebogen. Die Verwendung von metallischen Implantaten beeinträchtigt allerdings die Wahl von Röntgen- oder MRI-Diagnostik der Implantatstelle.

Die Patentanmeldung US 2019069984 A1 beschreibt ein Polymer-basierendes Implantat zum Ersatz eines Knochens, wobei ein poröser Körper mit einem nicht porösen Material verbunden ist, sodass das Implantat eine poröse Fläche und eine nicht-poröse Fläche aufweist. Die dabei verwendeten Werkstoffe und Schichtdicken sind dabei so gewählt, dass das Implantat intraoperativ in die gewünschte Form gebracht werden kann, sodass auf die Verwendung einer metallischen Stützstruktur verzichtet werden kann. Durch ein solches Polymer-basierendes Implantat kann Röntgenstrahlung oder MRI zur Diagnostik der Implantatstelle verwendet werden.

Die intraoperative Anpassung der Implantatgeometrie beschränkt jedoch die mechanische Belastbarkeit des Implantats. Es ist daher eine erste Aufgabe der Erfindung, ein Implantat bereitzustellen, welches einerseits die Verwendung von Röntgenstrahlen oder MRI zur Diagnostik der Implantatstelle so wenig wie möglich beeinträchtigt, und sich andererseits durch eine hohe mechanische Belastbarkeit auszeichnet. Eine weitere Aufgabe ist es, ein Verfahren zur Fertigung eines solchen Implantats bereitzustellen.

Die erste Aufgabe wird gelöst durch die Merkmale des Patentanspruchs 1. Die weitere Aufgabe wird gelöst durch die Merkmale des Patentanspruchs 16. Vorteilhafte Ausführungen ergeben sich aus den abhängigen Patentansprüchen, der Beschreibung sowie aus den Figuren.

Zur Lösung der ersten Aufgabe wird ein Implantat zum Ersatz oder Auffüllen einer Fehlstelle eines flächenartigen Knochens vorgeschlagen. Das Implantat weist eine flächenartige Form mit einer ersten Seite und einer gegenüberliegenden zweiten Seite auf. Unter einer "flächenartigen Form" ist sowohl eine plane als auch eine gekrümmte Fläche zu verstehen. Das Implantat weist zumindest eine erste Schicht und eine zweite Schicht auf. Die erste Schicht besteht vollständig oder zu einem überwiegenden Anteil aus nicht-porösem Polyetheretherketon (PEEK) oder Polyethylen (PE), und weist eine solche Schichtdicke auf, dass die Form des Implantats durch die Form der ersten Schicht festgelegt ist. Unter "festgelegte Form" wird hierbei verstanden, dass das Implantat nicht zerstörungsfrei plastisch verformt werden kann. Die Form des Implantats kann daher intraoperativ durch Biegen nicht angepasst werden. Die zweite Schicht besteht vollständig oder zu einem überwiegenden Anteil aus einem porösen Polymer, welches kein Polyaryletherketon oder Polyetheretherketon ist. Die zweite Schicht ist auf der ersten Seite des Implantats angeordnet.

Besteht die erste Schicht vollständig oder zu einem überwiegenden Anteil aus nicht-porösem Polyethylen, so wird hierzu vorzugsweise ultrahochmolekulargewichtiges Polyethylen (UHMWPE) als Werkstoff gewählt.

Der erfindungsgemäße schichtförmige Aufbau des Implantats ermöglicht eine hohe mechanische Belastbarkeit, welche im Wesentlichen durch die nicht-poröse Schicht bereitgestellt wird. Durch die zweite Schicht aus einem porösen Polymer wird das Einwachsverhalten des Implantats in umliegendes Gewebe gewährleistet. Aufgrund der formstabilen ersten Schicht besteht keine Gefahr, dass die poröse zweite Schicht während des Implantationsvorgangs durch mechanische Überbelastung beschädigt wird.

Vorzugsweise weist das Implantat keine metallische Stützstruktur auf. Durch das völlige Weglassen einer solchen metallischen Stützstruktur ist eine Diagnostik der Implantatstelle mittels Röntgenstrahlen oder MRI völlig unbeeinträchtigt.

Vorzugsweise besteht die zweite Schicht vollständig oder zu einem überwiegenden Anteil aus einem der folgenden Werkstoffe: Polyethylen, Polyphenylensulfon oder Polypropylen, besonders bevorzugt aus ultrahochmolekulargewichtigem Polyethylen (UHMWPE). Diese Werkstoffe zeichnen sich durch eine hohe Biokompatibilität aus, und bieten in poröser Form ein gutes Einwachsverhalten für umliegendes Gewebe.

Vorzugsweise liegt die zweite Schicht in einer gepressten und verschmolzenen Granulatform vor. Dadurch wird eine homogene Porosität bei gleichzeitig hoher Widerstandsfähigkeit gegen oberflächliche Verletzungen der zweiten Schicht erzielt. Eine solche gepresste und verschmolzene Granulatform kann beispielsweise durch Verwendung einer Mischung von Ausgangspartikeln erzielt werden, bei der 10% des Gewichtsanteils eine Partikelgröße kleiner als 1000 Mikrometer aufweist, 50% des Gewichtsanteils eine Partikelgröße kleiner als 500 Mikrometer aufweist, und 90% des Gewichtsanteils eine Partikelgröße kleiner als 450 Mikrometer aufweist.

Gemäß einer bevorzugten ersten Ausgestaltung ist auf der zweiten Seite des Implantats keine weitere Schicht angeordnet, sodass die erste Schicht aus dem nicht-porösem Polyetheretherketon (PEEK) vollständig freiliegt. Diese Ausgestaltung ist besonders dann vorteilhaft, wenn auf der zweiten Seite des Implantats kein Gewebe anwachsen soll. Dies kann beispielsweise bei einem Schädeldach-Implantat gefordert sein. Bei einer solchen Schädeldach-Anwendung bildet daher vorzugsweise die zweite Seite des Implantats die proximale Seite.

Gemäß einer bevorzugten zweiten Ausgestaltung weist das Implantat auf der zweiten Seite eine dritte Schicht auf, welche aus demselben Werkstoff besteht wie die auf der ersten Seite des Implantats angeordnete zweite Schicht. In anderen Worten weist das Implantat in dieser Ausgestaltung eine mittlere erste Schicht aus nicht-porösem Polyetheretherketon (PEEK) oder Polyethylen (PE) auf, auf der zumindest abschnittsweise auf beiden Seiten je eine poröse Schicht angeordnet ist.. Diese Ausgestaltung ist dann vorteilhaft, wenn auf beiden Seiten des Implantats ein Anwachsen von Gewebe gewünscht ist, beispielsweise bei einem Orbita-Boden-Implantat.

Vorzugsweise unterscheidet sich eine Porosität und/oder eine Oberflächenrauigkeit der dritten Schicht von einer Porosität und/oder eine Oberflächenrauigkeit der zweiten Schicht. Dadurch kann das Einwachsverhalten von Gewebe in die zweite und dritte Schicht gezielt beeinflusst werden. So verbessert eine mikroporöse Struktur das Einwachsverhalten von Bindegewebe, während eine makroporöse Struktur das Einwachsen von Hartgewebe wie beispielsweise Knochenzellen fördert. Die unterschiedliche Porosität kann beispielsweise durch die Wahl der Ausgangspartikelgröße erzielt werden. So kann für die Herstellung der zweiten Schicht beispielsweise eine Ausgangspartikelgröße mit maximal 1000 Mikrometer verwendet werden, und für die Herstellung der dritten Schicht eine Ausgangspartikelgröße mit maximal 700 Mikrometer. Alternativ dazu kann die zweite und dritte Schicht die gleiche Porosität und/oder Oberflächenrauigkeit aufweisen. Ist das Implantat als Orbita-Boden-Implantat ausgeführt, so kann beispielsweise für die dritte Schicht, welche die Unterseite des Implantats bildet, ein grobkörnigeres Granulat verwendet werden als für die zweiten Schicht, welche für die dem Augapfel zugewandte Oberseite des Implantats bildet.

Vorzugsweise ist zumindest ein Abschnitt der ersten Schicht von keiner weiteren Schicht bedeckt. In anderen Worten liegt das nicht-poröse Polyetheretherketon (PEEK) oder Polyethylen (PE) teilweise frei, sodass in diesem freiliegenden Bereich das Einwachsen von Gewebe gezielt verhindert werden kann.

Die erste Schicht aus nicht-porösem Polyetheretherketon (PEEK) oder Polyethylen (PE) ist vorzugsweise entweder vollflächig ausgebildet oder durch mehrere miteinander verbundene, aber abschnittsweise voneinander getrennte Bahnen gebildet. So kann die erste Schicht beispielsweise einen umlaufenden Rand aufweisen, welcher mehrere voneinander getrennte Bahnen umgibt, wobei die Bahnen und der umlaufende Rand einteilig ausgebildet sind. Die zweite Schicht aus dem porösen Polymer kann sich bei einer solchen Ausgestaltung auch über die Zwischenräume zwischen den Bahnen erstrecken, sodass die zweite Schicht abschnittsweise auf beiden Seiten freiliegt. Durch eine solche Ausbildung kann das Implantat so ausgeführt werden, dass auf beiden Seiten des Implantats zumindest an definierten Stellen Weichgewebe an das Implantat anwachsen kann, ohne dass auf beiden Seiten der ersten Schicht eine poröse Schicht aufgebracht werden muss. Dadurch kann die Menge des in den Körper implantierten Fremdmaterials gering gehalten werden.

Vorzugsweise weist die erste Schicht aus nicht-porösem Polyetheretherketon zumindest eine Durchgangsöffnung auf. Dadurch wird im implantierten Zustand ein Flüssigkeitsaustausch sowie ein Druckausgleich zwischen der ersten Seite und der zweiten Seite ermöglicht.

Vorzugsweise weist das Implantat zumindest eine Ausnehmung zur Aufnahme eines Befestigungselements auf. Die Ausnehmung ist in der ersten und/oder in der zweiten Schicht ausgebildet. Das Befestigungselement kann beispielsweise durch eine metallische Platte gebildet sein, welche vorzugsweise aus Titan oder Edelstahl gefertigt ist. Das Implantat kann bei einer solchen Ausführung mittels Schrauben an der umliegenden Anatomie befestigt werden. Alternativ oder ergänzend dazu kann das Befestigungselement als Nahtanker ausgeführt sein, sodass das Implantat an die umliegende Anatomie angenäht werden kann.

Vorzugsweise ist zumindest eine der Schichten mit Elementen aus zumindest einem der folgenden Materialen angereichert: Silber, Strontium, Magnesium, Tricalciumphosphat, Hydroxylapatit, Molybdän, Calciumcarbonat. Durch die antibakterielle und bioaktive Wirkung dieser Stoffe wird eine Entzündungswahrscheinlichkeit nach dem Implantationsvorgang verringert, bzw. das Einwachsverhalten in umliegendes Gewebe verbessert.

Gemäß einer bevorzugten Ausgestaltung bildet das Implantat ein Schädeldach-Implantat. Bei einer solchen Ausgestaltung ist es vorteilhaft, wenn die zweite Seite des Implantats die proximale Seite bildet, also die im implantierten Zustand der Dura zugewandte Seite. Bei einer solchen Ausführung ist es ferner vorteilhaft, wenn die erste Seite bis auf, falls vorhanden, Ausnehmungen zur Aufnahme von Befestigungselementen, vollflächig mit der Schicht aus dem porösen Polymer bedeckt ist. Die zweite, der Dura zugewandten Seite ist bei einer solchen Ausführung vorzugsweise von keiner weiteren Schicht bedeckt, sodass das nicht-poröse Polyetheretherketon auf der zweiten Seite des Implantats freiliegt.

Gemäß einer weiteren bevorzugten Ausgestaltung bildet das Implantat ein Augenhöhlen-Implantat, welches auch als Orbita-Implantat bezeichnet wird. Bei einer solchen Ausgestaltung ist es vorteilhaft, wenn die zweite Seite des Implantats jene Seite bildet, die im implantierten Zustand dem Augapfel zugewandt ist. Auf jenem Abschnitt, auf dem der Augapfel im implantierten Zustand gleiten soll, liegt entweder das nicht-poröse Polyetheretherketon oder Polyethylen frei, oder es ist eine dritte mikroporöse Schicht vorgesehen. Auf der ersten Seite des Implantats ist zumindest abschnittsweise die zweite Schicht aus dem porösen Polymer aufgebracht, sodass auf dieser Seite Gewebe in das Implantat einwachsen kann.

Zur Lösung der weiteren Aufgabe wird ein Verfahren zur Herstellung eines Implantats vorgeschlagen, welches im gefertigten Zustand zumindest die Merkmale des Patentanspruchs 1 aufweist. Das Verfahren ist durch folgende Schritte gekennzeichnet: Bereitstellen der fertig geformten ersten Schicht aus dem nicht-porösen Polyetheretherketon (PEEK) oder Polyethylen (PE), Aktivieren zumindest eines Teils der Oberfläche der ersten Schicht mit Niederdruck-Plasma, und anschließendes Aufbringen der zweiten Schicht auf die entsprechend aktivierte Oberfläche der ersten Schicht durch Aufpressen und Verschmelzen eines Granulats.

Durch die Aktivierung der Oberfläche der ersten Schicht wird die Haftung der zweiten Schicht auf der ersten Schicht deutlich verbessert. Die konkrete Konfiguration des Niederdruckplasmas hängt von der Geometrie der ersten Schicht ab, wobei sich in Versuchen eine aufgebrachte maximale Leistung von 100-400 Watt bewährt hat. Vorzugsweise erfolgt der Aufpressvorgang der zweiten Schicht zeitlich unmittelbar auf die Oberflächenaktivierung der ersten Schicht, vorzugsweise mittels einer Matrize und einem Stempel, deren Formgebung die Implantatgeometrie vorgibt. Bei dem Aufpressvorgang sind Druck und Temperaturverlauf über der Zeit vorzugsweise vorgegeben, sodass ein reproduzierbarer Prozess gebildet wird. Die individuellen Werte für Druck und Temperatur über der Zeit hängen von der Implantatgeometrie ab.

Ausführungsbeispiele der Erfindung sind anhand der Figuren detailliert beschrieben. Es zeigen:
- Fig. 1 und Fig. 2: zeigen je eine Ansicht eines Implantats gemäß einem ersten Ausführungsbeispiel;
- Fig. 3 und Fig. 4: zeigen je eine Detailansicht des Implantats gemäß dem ersten Ausführungsbeispiel;
- Fig. 5: zeigt eine schematische Seitenansicht eines Implantats gemäß einem zweiten Ausführungsbeispiel;
- Fig. 6: zeigt eine schematische isometrische Ansicht eines Implantats gemäß einem dritten Ausführungsbeispiel;
- Fig. 7: zeigt eine schematische Draufsicht eines Implantats gemäß einem vierten Ausführungsbeispiel;
- Fig. 8: zeigt eine schematische Draufsicht eines Implantats gemäß einem fünften Ausführungsbeispiel; und
- Fig. 9: zeigt eine schematische Draufsicht eines Implantats gemäß einem sechsten Ausführungsbeispiel.

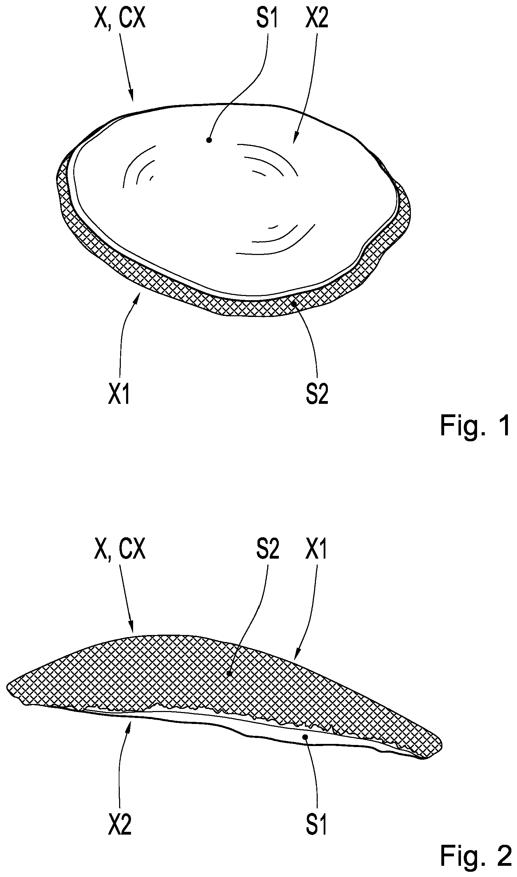

Fig. 1 und Fig. 2 zeigen je eine Ansicht eines Implantats X gemäß einem ersten Ausführungsbeispiel. Das Implantat X ist dazu geeignet, eine Fehlstelle eines flächenartigen Knochens zu ersetzen oder aufzufüllen. Das in Fig. 1 und Fig. 2 dargestellte erste Ausführungsbeispiel des Implantats X ist für die Anwendung im Cranium vorgesehen, beispielsweise als Schädeldach-Implantat CX. Das Implantat X weist eine flächenartige gekrümmte Form mit einer ersten Seite X1 und einer gegenüberliegenden zweiten Seite X2 auf. Das Implantat X weist eine erste Schicht S1 aus einem nicht-porösem Polyetheretherketon oder nicht-porösem Polyethylen auf, vorzugsweise aus ultrahochmolekulargewichtiges Polyethylen. Auf der ersten Seite X1 weist das Implantat X eine zweite Schicht S2 aus einem porösen Polymer auf, welche auf der ersten Schicht S1 angebracht ist. Die zweite Schicht S2 besteht vollständig oder zu einem überwiegenden Anteil aus Polyethylen, Polyphenylensulfon oder Polypropylen, besonders bevorzugt aus ultrahochmolekulargewichtiges Polyethylen (UHMWPE). Die erste Schicht S1 ist dabei so dick, dass eine intraoperative Formgebung des Implantats X nicht möglich ist. In anderen Worten ist die erste nicht-poröse Schicht S1 so dick ausgeführt, dass diese formstabil ist, sodass eine plastische Verformung der ersten Schicht S1 zur Anpassung der Implantatgeometrie nicht möglich ist, ohne die zweite poröse Schicht S2 zu beschädigen. Die für dieses mechanische Verhalten zumindest erforderliche Wandstärke der ersten Schicht S1 hängt von der Größe und Formgebung des Implantats X ab. Im in Fig. 1 dargestellten Beispiel ist das Implantat X etwa 10 Zentimeter lang und 10 Zentimeter breit, und weist eine gekrümmte Flächenform auf. Ist die erste Schicht S1 aus Polyetheretherketon oder ultrahochmolekulargewichtigen Polyethylen gefertigt, so beträgt die Wandstärke der ersten Schicht S1 bei dieser Geometrie des Implantats X im Mittel 2 bis 4 Millimeter. Abhängig von der mechanischen Belastung des Implantats X kann die erste Schicht S1 auch eine größere mittlere Wandstärke aufweisen, beispielsweise 6 Millimeter.

Die zweite Schicht S2 liegt in gepresster und verschmolzener Granulatform vor. Dadurch wird eine homogene Porosität der zweiten Schicht S2 bei gleichzeitig hoher Widerstandsfähigkeit gegen oberflächliche Verletzungen erzielt. Durch die homogene Porosität wird ein Einwachsen von Gewebe in die zweite Schicht S2 ermöglicht, während durch die nicht-poröse Ausgestaltung der ersten Schicht S1 ein Einwachsen von Gewebe in die erste Schicht S1 verhindert wird.

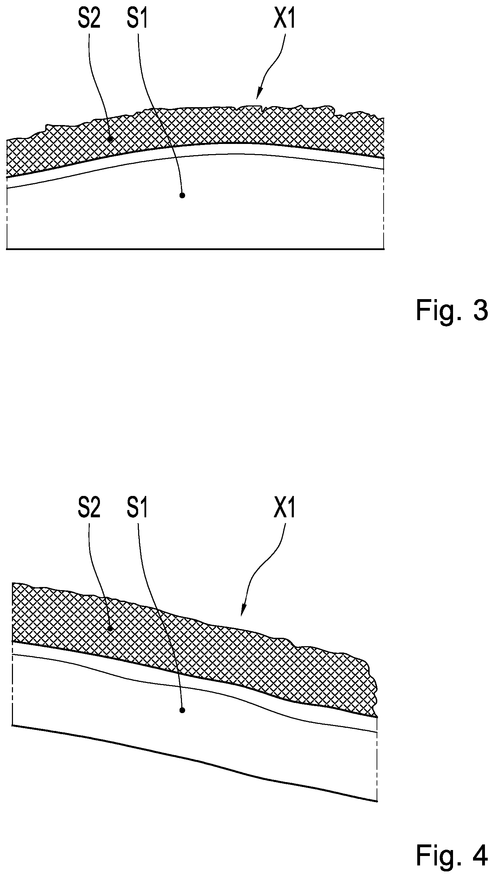

Fig. 3 und Fig. 4 zeigen je eine Detailansicht des Implantats X gemäß dem ersten Ausführungsbeispiel. Zur Herstellung eines derartigen Implantats X wird zunächst die erste Schicht S1 hergestellt, beispielsweise durch eine Formpresse oder durch ein generatives Fertigungsverfahren, auch bekannt als 3D-Druck. Insbesondere durch Nutzung eines generativen Fertigungsverfahrens ist eine Herstellung der ersten Schicht S1 genau nach den individuellen Bedürfnissen eines Patienten möglich. Anschließend wird die erste Schicht S1 gegebenenfalls nachbearbeitet, um Grate oder Stützstrukturen des vorhergehenden Fertigungsschritts zu entfernen. Anschließend wird zumindest ein Teil der ersten Schicht S1 durch Nutzung eines Niederdruck-Plasmas aktiviert, um eine besonders haftfähige Oberfläche zu erzeugen. Unmittelbar anschließend wird die zweite Schicht S2 auf die zuvor aktivierte Oberfläche der ersten Schicht S1 aufgebracht, und zwar durch Aufpressen und Verschmelzen eines Granulats. Auf diese Weise kann die zweite Schicht S2 sicher auf die erste Schicht S1 aufgebracht werden, ohne dass ein zusätzliches Haftmittel oder ähnliches erforderlich ist.

Aus den Darstellung gemäß Fig. 3 wird deutlich, dass die zweite Schicht S2 erheblich dünner sein kann als die erste Schicht S1. Fig. 4 zeigt eine Ausführung, bei der die zweite Schicht S2 ähnlich dick ist wie die erste Schicht S1. Die konkrete Dicke der porösen zweiten Schicht S2 kann abhängig von Anwendungszweck und spezifischen Bedürfnissen des Patienten gewählt werden. Die zweite Schicht S2 kann eine inhomogene Schichtdicke aufweisen, sodass an einer Stelle des Implantats X eine dickere zweite Schicht S2 vorliegt als an einer anderen Stelle. In Versuchen hat sich eine Dicke der zweiten Schicht S2 zwischen 1 und 6 Millimeter bewährt. Eine typische Gesamtdicke des Implantats X mit einem zweischichtigen Aufbau aus erster Schicht S1 und zweiter Schicht S2 beträgt 4 bis 8 Millimeter.

Die Darstellung der porösen zweiten Schicht S2 in den Figuren ist abstrahiert, da eine Darstellung der tatsächlichen Porosität in Patentzeichnungen nicht zuverlässig erkennbar und reproduzierbar ist.

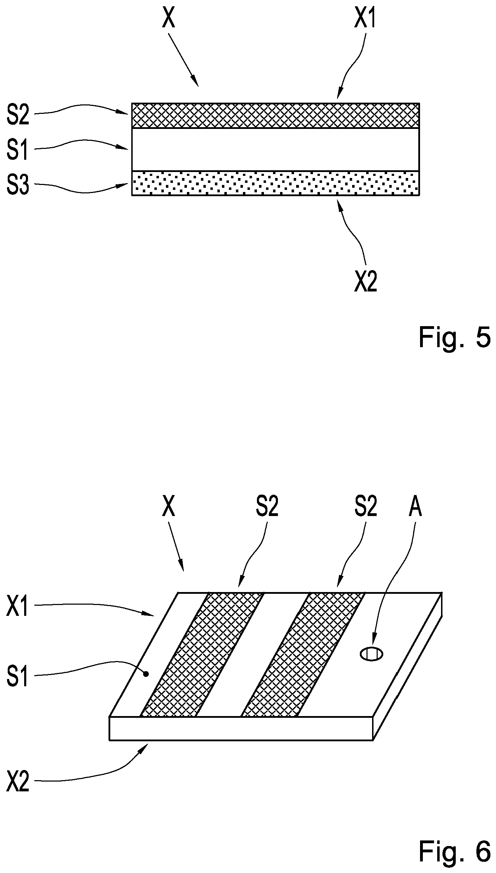

Fig. 5 zeigt eine schematische Seitenansicht eines Implantats X gemäß einem zweiten Ausführungsbeispiel. In dieser Ausgestaltung weist das Implantat X sowohl auf der ersten Seite X1 als auch auf der gegenüberliegenden zweiten Seite X2 eine poröse Schicht auf. Dazu ist auf der ersten Seite X1 die zweite Schicht S2 angeordnet, während auf der zweiten Seite X2 eine dritte Schicht S3 angeordnet ist. Die dritte Schicht S3 ist dabei aus demselben Werkstoff ausgebildet wie die zweite Schicht S2. Allerdings weist die dritte Schicht S3 eine andere Porosität auf als die zweite Schicht S2, wie in der Darstellung gemäß Fig. 5 angedeutet ist. Die unterschiedliche Porosität wird durch Verwendung einer unterschiedlichen Granulatgröße erzielt. Die erste Schicht S1 liegt zwischen der zweiten Schicht S2 und der dritten Schicht S3, sodass das Implantat X einen dreischichtigen Sandwichaufbau aufweist.

Fig. 6 zeigt eine schematische isometrische Ansicht eines Implantats X gemäß einem dritten Ausführungsbeispiel. In dieser Ausgestaltung sind mehrere Abschnitte der ersten Schicht S1 von keiner weiteren Schicht bedeckt, sodass Abschnitte der ersten Schicht S1 auf beiden Seiten X1, X2 des Implantats X freiliegen. Auf der ersten Seite X1 sind lediglich zwei Bahnen der zweiten Schicht S2 auf der ersten Schicht S1 aufgebracht. Die bahnen-förmig angeordnete zweite Schicht S2 ist dünnwandig ausgebildet, beispielsweise mit einer Schichtdicke von nur einem Millimeter. In einem beidseitig unbedeckten Abschnitt der ersten Schicht S1 ist eine Durchgangsöffnung A vorgesehen. Durch die Durchgangsöffnung wird im implantierten Zustand ein Flüssigkeitsaustausch sowie ein Druckausgleich zwischen der ersten Seite X1 und der zweiten Seite X2 ermöglicht. Das Implantat X kann mehrere derartige Durchgangsöffnungen A aufweisen.

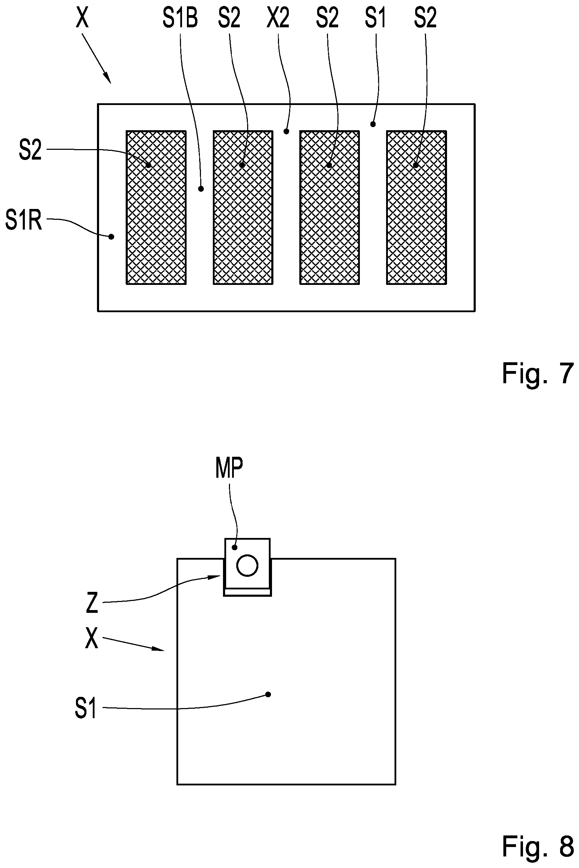

Fig. 7 zeigt eine schematische Draufsicht auf die zweite Seite X2 eines Implantats X gemäß einem vierten Ausführungsbeispiel. In dieser Ausgestaltung weist die erste Schicht S1 einen umlaufenden Rand S1R und mehrere miteinander verbundene Bahnen S1B auf. Zwischen den Bahnen S1B weist die erste Schicht S1 mehrere Ausnehmungen auf. Im Gegensatz zu den in Fig. 1 bis Fig. 6 dargestellten Ausführungsbeispielen ist die erste Schicht S1 daher nicht vollflächig ausgebildet. Auf der in Fig. 7 nicht sichtbaren ersten Seite X1 des Implantats X ist die zweite Schicht S2 angeordnet, welche durch die Ausnehmungen der ersten Schicht S1 auch von der zweiten Seite X2 zu sehen ist. In dieser Ausgestaltung ist die zweite Schicht S2 daher abschnittsweise beidseitig zugänglich. Bei einer derartigen Ausgestaltung kann auf der zweiten Seite X2 zumindest abschnittsweise eine dritte Schicht S3 aus einem porösen Polymer angeordnet sein, so wie in Fig. 5 schematisch dargestellt. Aus Gründen der Übersichtlichkeit ist eine solche Variante in den Figuren nicht explizit dargestellt.

Fig. 8 zeigt eine schematische Draufsicht eines Implantats X gemäß einem fünften Ausführungsbeispiel. Am Rand des Implantats X ist eine Ausnehmung Z vorgesehen, welche zur Aufnahme eines Befestigungselements MP eingerichtet ist. Das Befestigungselement MP kann beispielsweise eine metallische Platte sein, welche ein Loch zur Aufnahme einer Befestigungsschraube oder einer Nahtschnur aufweist. Das Implantat X kann mehrere derartige Befestigungselement MP aufweisen, die in mehrere am Rand des Implantats X verteilte Ausnehmungen Z angeordnet sind.

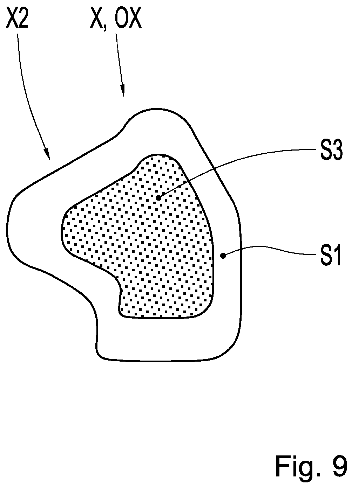

Fig. 9 zeigt eine schematische Draufsicht auf die zweite Seite X2 eines Implantats X gemäß einem sechsten Ausführungsbeispiel. Das Implantat X ist als Augenhöhlen-Implantat OX ausgebildet. Auf einem Abschnitt der zweiten Seite X2 ist eine dritte Schicht S3 aus einem porösen Polymer angeordnet. Am Randbereich des Implantats X liegt die nicht-poröse erste Schicht S1 frei, ist also von keiner porösen Schicht bedeckt. Auf der in Fig. 9 nicht sichtbaren ersten Seite X1 ist zumindest abschnittsweise die zweite Schicht S2 ausgebildet.

### Bezugszeichenliste

- X: Implantat
- CX: Schädeldachimplantat
- OX: Augenhöhlenimplantat
- X1: Erste Seite
- X2: Zweite Seite
- S1: Erste Schicht
- S1B: Bahn
- S1R: Rand
- S2: Zweite Schicht
- S3: Dritte Schicht
- A: Durchgangsöffnung
- Z: Ausnehmung
- MP: Befestigungselement

## Patentansprüche

1. Implantat (X) zum Ersatz oder Auffüllen einer Fehlstelle eines flächenartigen Knochens,
- wobei das Implantat (X) eine flächenartige Form mit einer ersten Seite (X1) und einer gegenüberliegenden zweiten Seite (X2) aufweist,
- wobei das Implantat (X) eine erste Schicht (S1) aus nicht-porösem Polyetheretherketon oder Polyethylen aufweist, welche eine solche Schichtdicke aufweist, dass die Form des Implantats (X) durch die Form der ersten Schicht (S1) festgelegt ist,
- wobei das Implantat (X) auf der ersten Seite (X1) zumindest abschnittsweise eine zweite Schicht (S2) aus einem porösen Polymer aufweist, welches kein Polyaryletherketon oder Polyetheretherketon ist.

2. Implantat (X) nach Anspruch 1, **dadurch gekennzeichnet, dass** das Implantat (X) keine metallische Stützstruktur aufweist.

3. Implantat (X) nach Anspruch 1 oder Anspruch 2, **dadurch gekennzeichnet, dass** die zweite Schicht (S2) vollständig oder zu einem überwiegenden Anteil aus einem der folgenden Werkstoffe besteht: Polyethylen, Polyphenylensulfon oder Polypropylen.

4. Implantat (X) nach Anspruch 3, **dadurch gekennzeichnet, dass** die erste Schicht (S1) und/oder die zweite Schicht (S2) aus ultrahochmolekulargewichtigem Polyethylen besteht.

5. Implantat (X) nach einem der Ansprüche 1 bis 4, **dadurch gekennzeichnet, dass** die zweite Schicht (S2) in gepresster und verschmolzener Granulatform vorliegt.

6. Implantat (X) nach einem der Ansprüche 1 bis 5, **dadurch gekennzeichnet, dass** auf der zweiten Seite (X2) des Implantats (X) keine weitere Schicht angeordnet ist, sodass die erste Schicht (S1) vollständig freiliegt.

7. Implantat (X) nach einem der Ansprüche 1 bis 5, **dadurch gekennzeichnet, dass** das Implantat (X) auf der zweiten Seite (X2) eine dritte Schicht (S3) aufweist, welche aus demselben Werkstoff besteht wie die zweite Schicht (S2).

8. Implantat (X) nach Anspruch 7, **dadurch gekennzeichnet, dass** sich eine Porosität und/oder Oberflächenrauigkeit der dritten Schicht (S3) von einer Porosität und/oder Oberflächenrauigkeit der zweiten Schicht (S2) unterscheidet.

9. Implantat (X) nach einem der Ansprüche 1 bis 8, **dadurch gekennzeichnet, dass** zumindest ein Abschnitt der ersten Schicht (S1) von keiner weiteren Schicht (S2, S3) bedeckt ist.

10. Implantat (X) nach einem der Ansprüche 1 bis 9, **dadurch gekennzeichnet, dass** die erste Schicht (S1) vollflächig ausgebildet ist oder durch miteinander verbundene Bahnen (S1B) gebildet ist.

11. Implantat (X) nach einem der Ansprüche 1 bis 10, **dadurch gekennzeichnet, dass** die erste Schicht (S1) zumindest eine Durchgangsöffnung (A) aufweist.

12. Implantat (X) nach einem der Ansprüche 1 bis 11, **dadurch gekennzeichnet, dass** in der ersten Schicht (S1) und/oder in der zweiten Schicht (S2) zumindest eine Ausnehmung (Z) zur Aufnahme eines Befestigungselements (MP) ausgebildet ist.

13. Implantat (X) nach einem der Ansprüche 1 bis 12, **dadurch gekennzeichnet, dass** zumindest eine der Schichten (S1, S2, S3) mit Elementen aus zumindest einem der folgenden Materialen angereichert ist: Silber, Strontium, Magnesium, Tricalciumphosphat, Hydroxylapatit, Molybdän, Calciumcarbonat.

14. Schädeldach-Implantat (CX), **gekennzeichnet durch** die Merkmale eines der Ansprüche 1 bis 13.

15. Augenhöhlen-Implantat (OX), **gekennzeichnet durch** die Merkmale eines der Ansprüche 1 bis 13.

16. Verfahren zur Herstellung eines Implantats (X, CX, OX) nach einem der Ansprüche 1 bis 15, **gekennzeichnet durch** folgende Schritte:
- Bereitstellen der fertig geformten ersten Schicht (S1),
- Aktivieren zumindest eines Teils der Oberfläche der ersten Schicht (S1) mit Niederdruck-Plasma,
- Aufbringen der zweiten Schicht (S2) auf die aktivierte Oberfläche der ersten Schicht (S1) durch Aufpressen und Verschmelzen eines Granulats.
